# EUROPEAN PATENT SPECIFICATION

(11) **EP 1 877 570 B1**
(45) Date of publication and mention of the grant of the patent: **15.12.2010**
(21) Application number: 06733160.3
(22) Date of filing: 13.04.2006
(51) Int. Cl.: C12Q 1/04, C12Q 1/22

(54) **ANALYSING BREATH SAMPLES FOR PENTYLFURAN**
ANALYSE VON ATEMPROBE AUF PENTYLFURAN
ANALYSE DE LA TENEUR EN PENTYLFURAN DANS DES ÈCHANTILLONS D'AIR EXPIRÈ

(30) Priority: 19.04.2005 NZ 53951805
(43) Date of publication of application: 16.01.2008
(73) Proprietor: Agresearch Limited, Hamilton (NZ); SYFT technologies Limited, Middleton, Christchurch (NZ); The University of Otago, Dunedin (NZ)
(72) Inventor: Scotter, Jennifer M, Christchurch 8250 (NZ); Chambers, Stephen T, Christchurch, 8002 (NZ); Syhre, Mona, Christchurch 8002 (NZ)
(74) Representative: Wright, Simon Mark
(86) International application number: PCT/NZ2006/000071
(87) International publication number: WO 2006/112733

(56) References cited:
- WILKINS K., NIELSEN E.M., WOLKOFF P.: "Patterns in Volatile Organic Compounds in Dust from Moldy Buildings" INDOOR AIR, vol. 7, no. 2, July 1997 (1997-07), pages 128-134, XP002491239 Danemark
- FISCHER GUIDO ET AL: "Relevance of airborne fungi and their secondary metabolites for environmental, occupational and indoor hygiene." ARCHIVES OF MICROBIOLOGY, vol. 179, no. 2, January 2003 (2003-01), pages 75-82, XP002491217 ISSN: 0302-8933
- GAO P. ET AL.: 'Determination of unique microbial volatile organic compounds produced by five Aspergilus species commonly found in problem buildings' AIHA JOURNAL vol. 63, no. 2, 2002, pages 135 - 140, XP009091499
- DE LACY COSTELLO B.P.J. ET AL.: 'Gas chromatography-mass spectrometry analyses of volatile organic compounds from potato tubers inoculated with Phytophora infestans or Fusarium coeruleum' PLANT PATHALOGY vol. 50, August 2001, pages 489 - 496, XP003020072
- GUERMION N. ET AL: 'Identifying bacteria in human urine: current practice and the potential for rapid, near-patient diagnosis by sensing volatile organic compounds' CLINICAL CHEMISTRY AND LABORATORY MEDICINE vol. 39, no. 10, 2001, pages 893 - 906, XP001061839

## Description

### Field of the Invention

The invention relates to the determination of a unique biomarker pentylfuran and to its use to test for fungal and bacterial pathogens, including *Aspergillus fumigatus* in an individual or from a culture using analysis of breath/ samples.

### Background to the invention

All micro-organisms produce by-products as a result of their normal metabolism. The ability of different organisms to metabolise different substrates in order to satisfy their energy and nutritional requirements is fundamental to laboratory microbiology, and forms the basis of many rapid identification tests. The metabolites by a single species can vary widely, depending upon the growth substrate, conditions (temperature, oxygen availability), and the age of the culture itself.
Amongst the many large primary and secondary metabolites produced by microbes, some organic substances are formed which readily volatilise at low temperatures. Microbial volatile organic compounds (MVOCs) have been studied extensively in agriculture and food production, as some MVOCs have important health and economic implications in these fields. For example, some MVOCs have been associated with spoilage in stored crops and foodstuffs, where they may be responsibly for tainted, "off' flavours, discolouring of products, or toxicity. Profiles of MVOCs are increasingly being found to be unique to the species or strain level.

Invasive aspergillosis is one of the most problematic infections due to difficulties of diagnosis and treatment. Volatile organic compounds (VOCs) have the potential to improve the specificity and sensitivity of diagnosis of this and other infections. It would be useful to identify a unique biomarker of *Aspergillus* species, particularly *Aspergillus fumigatus,* in the headspace gas of *in vitro* cultures and to detect the marker from breath samples of infected or colonised patients.

### Object of the Invention

It is an object of the invention to provide a biomarker to detect bacterial and/or fungal pathogens such as *Aspergillus fumigatus* in a biosample or at least to provide the public with a useful choice.

### Summary of the Invention

The invention provides the biomarker, pentylfuran for the use in bio analysis of microorganisms such as fungal and bacterial pathogens in a gaseous sample from breath of an animal, including a human. In particular, the invention provides the use of the biomarker pentylfuran to detect fungal species, more particularly, *Aspergillus* species in a gaseous biosample from breath of an animal, including a human.

More particularly the invention provides the use of the biomarker pentylfuran to detect *Aspergillus fumigatus* in a gaseous biosample from breath of an animal, including a human.

The biosample may, most preferably, a breath sample of a patient.

In particular, the invention provides the use of the biomarker pentylfuran, in the bio analysis of microorganisms in a gaseous bio sample from bream of an animal, including a human.

Other micro organisms such as *Aspergillus flavus, Haemophilus influenza* and *Pseudomonas aeruginosa* may also be detected. The biosample is a breath sample. Preferably the biomarker is 2-pentylfuran. However it could be 3-pentylfuran.

The invention provides a ex-vivo method of detecting *Aspergillus* in a patient comprising:
(a) analysing a biosample obtained from the breath of the patient for the presence of pentylfuran; and
(b) determining whether *Aspergillus* is present in the breath sample.

The invention also provides the use of a biomarker pentylfuran in the detection of *Aspergillus fumigatus* from a breath sample of an animal.

The *Aspergillus* species is preferably *Aspergillus fumigatus*.

### Description of the Drawings

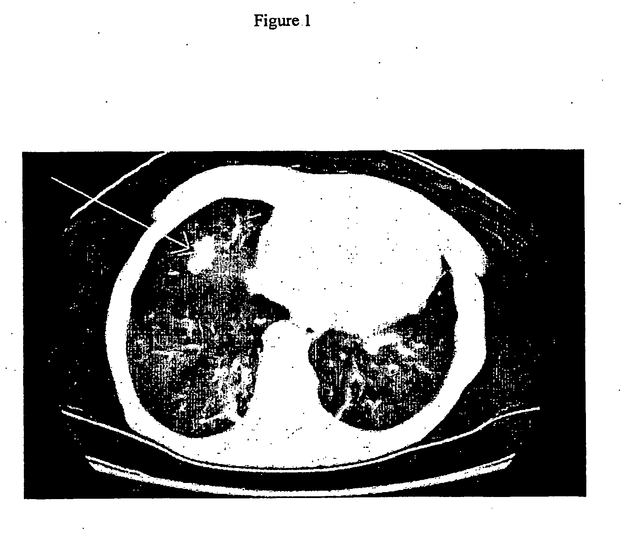

Figure 1 shows a chest CT scan of a patient with multiple foci of air space opacity surrounded by hyperdense maternal.

### Detailed Description of the Invention

The invention will now be described, by way of example only

Gas Chromatography-Mass Spectroscopy (GC-MS) combined with Solid Phase Micro Extractian (SPME) was used to identify pentylfuran as a specific biomarker of *A. fumigatus* from cultures. Four litre breath samples were collected from patients with Cystic Fibrosis, with or without colonisation of *A. fumigatus* and other pathogens, and healthy volunteers. Breath samples were semi-quantitatively analysed by SPME / GC-MS for presence or absence of pentylfuran.
A total of 21 individuals were tested. Pentylfuran was detected from breath samples of 4/4 patients with CF and *A. fumigatus* colonisation, 3/7 patients with CF and no microbiological evidence of *A. fumigatus* and 0/10 healthy control individuals.

### Materials and Methods

### Strains and culture conditions

Clinical isolates of *Aspergillus flavus, Aspergillus fumigatus, Candida albicans, Mucor racemosus, Fusarium solani,* and *Cryptococcus neoformans* were used in these experiments.
Organisms were grown on blood agar within 100 ml sterile glass vials stoppered with airtight aluminium caps incorporating a teflon-coated rubber septum.

### Strain preparation.

Strains were grown for 72 hours on blood agar plates, then, in, the case of yeast species, a sterile loop of culture was removed from the plate and transferred to 5 mL of sterile water. For filamentous fungal strains, spores were harvested from the plate with sterile water containing 0.05% Tween. Five hundred microlitres of this suspension was introduced to the sealed culture vial by injecting through the septum into the medium.
Cultures were maintained at 37°C for 5 days. Vials were flushed with 100 ml purified dry air once every approximately 12 hours.

### Detection of pentylfuran by GC-MS. Calibration and standardisation

Calibration curves of headspace gas analysis of serial aqueous dilutions of pentylfuran were plotted. The resulting calibration curve proved to be linear in the range 1-50 pg.

### Patient demographics

Four participants with cystic fibrosis and *Aspergillus* colonisation, 6 patients with CF and no *Aspergillus* colonisation, and 10 control individuals meeting the inclusion criteria for the study were identified. Relevant demographic and clinical data are shown in table 2.

### Soud-Phase Micro Extraction (SPME) Gas Chromatography - Mass Spectrometry (GC-MS).

A comprehensive literature review was performed, and a database of all reported MVOCs from *Aspergillus* species was created, which included the compound isolated, species and strain, culture medium and conditions, and analysis method. Headspace gases of strains cultured as described above were subjected to SPME / GC-MS analysis.

### Sample preparation

The conditioned SPME fibre was exposes into culture vials for 10 minutes and then desorbed directly in the injection port for 5 minutes.

### GC/MS parameters

The temperatures of the injector, ion trap, manifold and transfer line were 250,200, 60 and 250°C, respectively. The oven program commenced at 50°C for 2 minutes and was raised to 250°C at a rate of 10°C/min, at which temperature it was maintained for a further 2 minutes. Helium flow was set at a constant rate of 1.2 ml/min. The split vent was opened to a ratio of 1:50 after 1 minute. Fragmentation was performed in the EI-mode as full scan which gave additional certainty- Further MS/MS fragmentation could be used to further increase sensitivity.

### Calibration and semi-quantification

Fifty microlitres of diluted pentylfuran solutions in methanol were deposited into 20 mL headspace vials. The final calibration was made up as total amounts in the headspace vials containing 1,5,10 and 50 pg, respectively. The fibre was exposed into the headspace vials for 5 min using the Combi-PAL autosampler.

### Patient selection

Patients enrolled on the study included individuals with cystic fibrosis (CF) colonised chronically with aspergillus, patients with CF not colonised with aspergillus, and healthy control individuals. Ethical approval for the study was obtained from the local ethics committee, and participants gave their informed consent to take part in the study. Inclusion criteria for patients to act as "positive" (colonised with *Aspergillus*) were a history of positive culture for *A. fumigatus* from lower respiratory tract specimens (sputum, BAL, tracheal aspirate, or cough swab in cases where sputum had not been made available to the microbiology laboratory). Patients needed to have a minimum of three positive results within the past 12 months, and ideally one from within 1 month of testing. Patients were excluded from the study if they were currently undergoing treatment with itraconazole for ABPA.

Patients in the cystic fibrosis "uncolonised" group were selected if they had no current or past history of positive culture results for *Aspergillus* as described above, and no clinical evidence of aspergillus colonisation. Healthy control individuals were recruited from laboratory staff. These participants were asked to complete a questionnaire which served to give information concerning any recent antibiotic use or evidence of respiratory or urinary tract infection. Any participants not meeting these criteria were excluded from the study.

### Breath sampling

Breath samples were collected into a 4L tedlar bag, which incorporated a valve, disposable mouthpiece and septum that could be pierced for sampling. Samples were collected by asking participants to exhale through the mouth into the bag until full The valve in the bag was then closed, and samples transported immediately to the laboratory for testing.

### Analysis of breath samples by GC-MS

Breath samples were analysed by GC-MS for presence and quantity of pentylfuran as described for cultures above. The conditioned SPME fibre was exposed into the collection bags for 48h and then desorbed directly in the injection port for 5 minutes.

### Results

### Detection of pentylfuran from laboratory cultures.

The results for testing of laboratory strains for the presence of pentylfuran are given in Table I.

**Table 1. Results of screening fungal and bacterial isolates by GC-MS for presence of pentylfuran.**

| Organism | Source | pentylfuran |
|---|---|---|
| *A.flavus* | Clinical (ear swab) | nd |
| *A.flavus* | Clinical (ear swab) | nd |
| *A.niger* | Environmental | xx |
| *A.niger* | Environmental | xx |
| *F.oxysporum* | Environmental | x |
| *M.racemosus* | Clinical (sinus biopsy) | x |
| *C.albicans* | Clinical (urine) | x |
| *C.albicans* | Clinical (urine) | x |
| *A.fumigatus* | Clinical (lung biopsy) | xx |
| *A.fumigatus* | clinical (sputum) | xx |
| A*.fumigatus* | Clinical (sputum) | xx |
| *A*.*fumigatus* | Clinical (BAL) | xx |
| *A.fumigatus* | Clinical (sputum) | xx |
| *A*.*fumigatus* | Clinical (sputum) | xx |
| *A.fumigatus* | Clinical (sputum) | xx |
| *A.fumigatus* | Clinical (sputum) | xxx |
| *A.fumigatus* | Clinical (sputum) | xx |
| *A.fumigatus* | Clinical (ear swab) | xx |
| *A*.*fumigatus* | Environmental | xx |
| *A.fumigatus* | Environmental | xx |
| *A.fumigatus* | Environmental | xx |
| *A.fumigatus* | Environmental | xxx |
| *A.fumigatus* | Environmental | xxx |
| *A.fumigatus* | Environmental | xxx |
| *A*.*fumigatus* | Environmental | xxx |
| *A.fumigatus* | Environmental | xxx |
| *A.fumigatus* | Environmental | xx |
| *A.fumigatus* | Environmental | xx |
| *H.influenzae* | Clinical (sputum) | x |
| *B*.c*epacia* | Clinical (sputum) | x |
| *Ps.aeruginosa* | Clinical (sputum) | x |
| *Ps.aeruginosa* | Clinical (sputum) | x |
| *S.aureus* | Clinical (sputum) | nd |
| *S.aureus* | Clinical (sputum) | nd |

| | | |
|---|---|---|
| Source: Spt; sputum, BAL; bronchoalveolar lavage pentylfuran: nd; not detected, x; low/trace levels, xx; moderate levels; xxx; high levels. | | |

### Analysis of breath samples for presence of pentylfuran

Four litre breath samples were assayed for the presence and quantity of pentylfuran. Results are shown in table 2, along with relevant clinical and microbiological data.

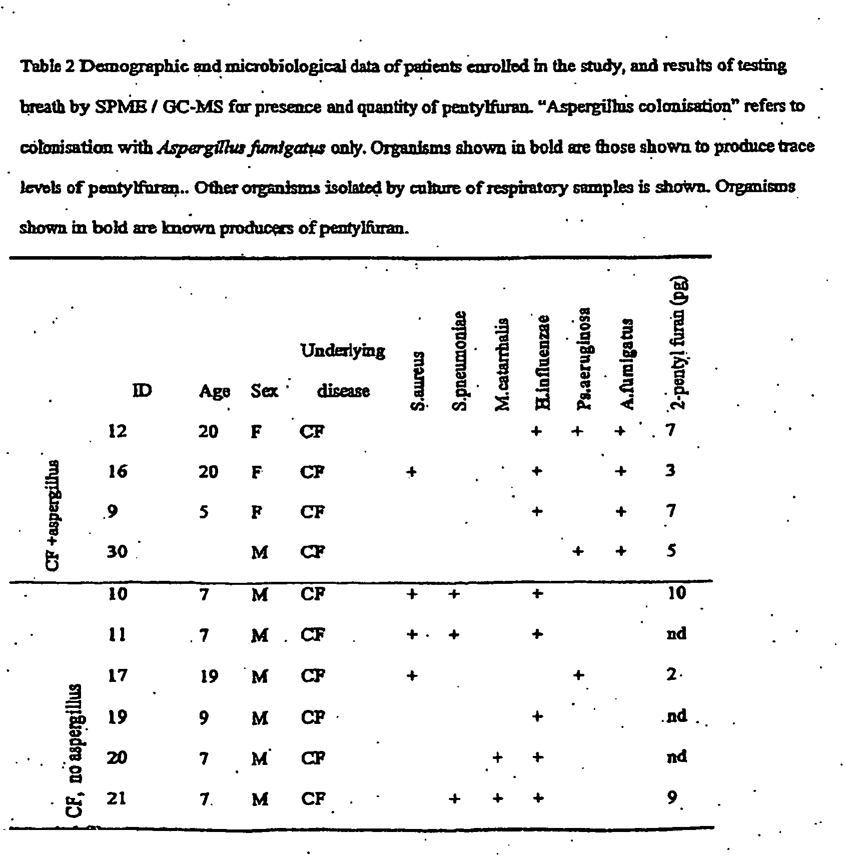

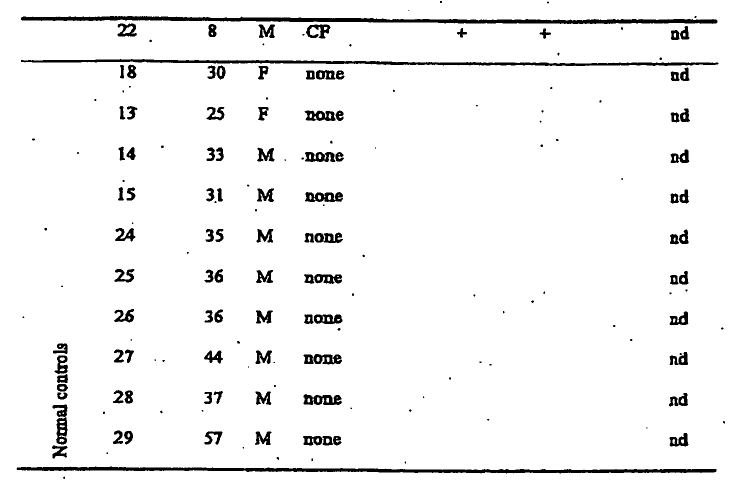

Pentylfuran was detected on the breath of all (n=4) patients colonised with *A.fumigatus.* Pentylfuran was also detected on breath samples of 3 of 7 patients without evidence of *Aspergillus* colonisation, but colonisation with other pathogens, including some shown to produce pentylfuran in modest quantities. Of the healthy individuals, one of ten subjects showed a trace level of pentylfuran on the breath.
The important message from this data set is that healthy, normal individuals appear not to produce pentylfuran (or only at baseline levels), while those with pulmonary colonisation with organisms that produce pentylfuran, do. This is the first time that the detection and quantification of a specific microbial metabolite from breath of infected/colonised individuals has been reported. Pentylfuran is a metabolite of *Aspergillus fumigatus,* and possibly other fungal and bacterial pathogens. These *in vitro* experiments showed that low levels of pentylfuran is produced by *A.flavus,* as well as *Pseudomonas aerugiviosa* and *Haemophi*/*us influerizae,* both of which are common colonisers of the longs of patients with CF.

Blood tests for *Aspergillus* infection are not optimal in that they are prone to sampling error, and may return false-negative results even in cases of proven disseminated infection. This may be further compounded by the administration of systemic antifungal therapy. Use of Broncho-alveloar lavage (BAL) for diagnostic purposes has had increasing support over recent years, notwithstanding that it is not an ideal procedure to be performing in a neutropenic, and often thrombocytopenic patient. However, BAL has the advantage over blood testing in that it allows direct sampling of the site of primary infection. Breath testing for metabolites of pathogens resident in the lungs may be viewed in a similar way to BAL, except that it is without the risks associated with BAL. The principle of breath testing, like BAL, is a more direct way of sampling the site of primary infection. DNA and antigen detection based assays will detect both viable and non-viable fungal cell elements. Detection of *A.fumigatus* metabolites from breath samples would suggest that the organism is metabolically active.
The results of this study show that:
1. Pentylfuran may be used as a biomarker of *Aspergillus fumigatus,* from cultures.
2. Pentylfuran can be detected from the breath of patients colonised or infected with *Aspergillus fumigatus* by GC-MS
3. Detection of pentylfuran from breath may form the basis of a useful diagnostic test for aspergillus infection.

### Case Report

A 79 year old woman undergoing treatment with dexamethasone and cyclophosphamide for multiple myeloma suffered an episode of febrile neutropenia (neutrophils 0.5-0.9 x 10⁹/L) that failed to resolve despite broad spectrum antibiotic therapy. A CT of the sinuses showed extensive disease suggestive of *Aspergillus* and the chest CT showed multiple foci of air space opacity with a surrounding halo of hyperdense material, suggestive of aspergillus infection (arrowed, figure 1). *A. fumigatus* was cultured from the sputum on 2 occasions. Nested PCR for *A. fumigatus* DNA in peripheral blood, and was negative on 6 occasions. The patient gave informed consent to participate in the study, and 3-litre breath samples were collected is described above for patients with CF. 2-pentylfuran was detected (10.1 pg) in the exhaled breath 2 days after the CT scan was performed. After treatment with voriconazole for 4 weeks, the lesions in the lungs had reduced in size, the breath test had become negative and aspergillus was no longer culturable from the sputum.

### Industrial Applicability

The invention will be of use in the medical area, assisting in the detection of microbes and pathogens in patients. In particular, the ability to detect *Aspergillus* species, especially *Aspergillus fumigatus* will be of use in detecting and hence treatment of the inflection caused by the microbe in patients.

## Claims

1. The use of the biomarker pentylfuran in the bioanalysis of microorganisms in a gaseous biosample from breath of an animal, including a human.

2. The use according to claim 1 wherein the microorganisms are fungal pathogens.

3. The use according to claim 1 or 2 wherein the microorganisms are from *Aspergillus species* or where the microorganism is an *Aspergillus* species.

4. The use according to any preceding claim wherein the microorganism is *Aspergillus fumigatus.*

5. The use according to any preceding claim wherein the pentylfuran is 2-pentylfuran.

6. An *ex vivo* method of detecting a bacterial or fungal pathogen; eg *Aspergillus* in patient, the method comprising:
a) analysing a biosample from the breath of the patient for the presence of pentylfuran; and
b) thus determining whether the pathogen is present in the breath biosample or in the patient.

7. An *ex vivo* method according to claim 6 wherein the *Aspergillus* species is *Aspergillus fumigatus.*

8. Use of the biomarker pentylfuran in the detection of a fungal or bacterial pathogen, such as *Aspergillus fumigatus,* from a breath sample of an animal.

9. Use according to claim 8 in which the animal is human.

10. The use of pentylfuran for the *ex vivo* detection of the presence of a microorganism; such as a bacterial or fungal pathogen in an animal; including a human where the pentylfuran is present in a gaseous biosample from the breath of the animal.

## Patentansprüche

1. Verwendung des Biomarkers Pentylfuran in der Bioanalyse von Mikroorganismen in einer gasförmigen Bioprobe aus dem Atem eines Tieres, einen Menschen einschließend.

2. Verwendung nach Anspruch 1, wobei die Mikroorganismen Pilzpathogene sind.

3. Verwendung nach Anspruch 1 oder 2, wobei die Mikroorganismen aus der *Aspergillus*-Spezies kommen oder wobei der Mikroorganismus eine *Aspergillus*-Spezies ist.

4. Verwendung nach einem der vorangehenden Ansprüche, wobei der Mikroorganismus *Aspergillus fumigatus* ist.

5. Verwendung nach einem der vorangehenden Ansprüche, wobei das Pentylfuran 2-Pentylfuran ist.

6. *Ex vivo* Verfahren zum Nachweis eines bakteriellen oder Pilzpathogens, z.B. *Aspergillus,* in einem Patienten, wobei das Verfahren umfasst, dass
a. eine Bioprobe aus dem Atem des Patienten auf das Vorhandensein von Pentylfuran hin analysiert wird; und
b. somit bestimmt wird, ob das Pathogen in der Atembioprobe oder in dem Patient vorhanden ist.

7. *Ex vivo* Verfahren nach Anspruch 6, wobei die *Aspergillus-Spezies Aspergillus fumigatus* ist.

8. Verwendung des Biomarkers Pentylfuran zum Nachweis eines Pilz- oder bakteriellen Pathogens, wie *Aspergillus fumigatus,* in der Atemprobe eines Tieres.

9. Verwendung nach Anspruch 8, wobei das Tier ein Mensch ist.

10. Verwendung von Pentylfuran zum *ex vivo* Nachweis des Vorhandenseins eines Mikroorganismus, wie eines bakteriellen oder Pilzpathogens, in einem Tier, einen Menschen einschließend, wobei das Pentylfuran in einer gasförmigen Bioprobe aus dem Atem des Tieres vorhanden ist.

## Revendications

1. Utilisation d'un biomarqueur pentyl-furane dans la bioanalyse de micro-organismes présents au sein d'un échantillon biologique gazeux provenant de l'haleine d'un animal, y compris un humain.

2. Utilisation conforme à la revendication 1, dans laquelle les micro-organismes sont des agents pathogènes fongiques.

3. Utilisation conforme à la revendication 1 ou 2, dans laquelle les micro-organismes sont choisis parmi les espèces d'*Aspergillus* ou le micro-organisme est une espèce d'*Aspergillus.*

4. Utilisation conforme à l'une des revendications précédentes, dans laquelle le micro-organisme est *Aspergillus fumigatus.*

5. Utilisation conforme à l'une des revendications précédentes, dans laquelle le pentyl-furane est du 2-pentyl-furane.

6. Procédé *ex vivo* de détection d'un agent pathogène fongique ou bactérien, par exemple d'un *Aspergillus,* chez un patient, lequel procédé comporte les étapes suivantes :
a) analyser un échantillon biologique provenant de l'haleine du patient, pour y rechercher la présence de pentyl-furane,
b) et déterminer par là si l'agent pathogène est présent dans l'échantillon biologique d'haleine ou chez le patient.

7. Procédé *ex vivo* conforme à la revendication 6, dans lequel l'espèce d'*Aspergillus* est *Aspergillus fumigalus.*

8. Utilisation d'un biomarqueur pentyl-furane dans la détection d'un agent pathogène fongique ou bactérien, comme *Aspergillus fumigatus,* dans un échantillon d'haleine d'un animal.

9. Utilisation conforme à la revendication 8, dans laquelle l'animal est un humain.

10. Utilisation d'un pentyl-furane dans la détection *ex vivo* de la présence d'un micro-organisme, comme un agent pathogène fongique ou bactérien, chez un animal, y compris un humain, dans laquelle le pentyl-furane est présent au sein d'un échantillon biologique gazeux provenant de l'haleine de l'animal.
